# EUROPEAN PATENT SPECIFICATION

(11) **EP 2 597 105 B1**
(45) Date of publication and mention of the grant of the patent: **06.01.2016**
(21) Application number: 13155928.8
(22) Date of filing: 10.01.2008
(51) Int. Cl.: C07K 17/08, C07K 14/515, C07K 1/107, C07K 1/18, A61K 47/48, A61K 38/18, A61P 35/00

(54) **A conjugate comprising angiostatin or its fragment, the method for producing the conjugate and use thereof**
Konjugat mit Angiostatin oder dessen Fragment, Verfahren zur Herstellung des Konjugats und Verwendung davon
Conjugué comprenant de l'angiostatine ou son fragment, procédé de production du conjugué et son utilisation

(30) Priority: 10.01.2007 CN 200710004558
(43) Date of publication of application: 29.05.2013
(62) Divisional of application: 08700623.5
(73) Proprietor: Protgen Ltd., Haidian District, Beijing (CN); Tsinghua University, Haidian District, Beijing 100084 (CN)
(72) Inventor: Luo, Yongzhang, 100084 Beijing (CN); Chang, Guodong, 100085 Beijing (CN); Yang, Shuling, 100085 Beijing (CN); Gao, Lei, 100085 Beijing (CN); Fu, Yan, 100084 Beijing (CN)
(74) Representative: Hoffmann Eitle

(56) References cited:
- WO-A2-02/26782
- US-A1- 2004 082 030
- MACDONALD NICHOLAS J ET AL: "The tumor-suppressing activity of Angiostatin protein resides within kringles 1 to 3", BIOCHEMICAL AND BIOPHYSICAL RESEARCH COMMUNICATIONS, ACADEMIC PRESS INC. ORLANDO, FL, US, vol. 264, no. 2, 22 October 1999 (1999-10-22), pages 469-477, XP002304620, ISSN: 0006-291X
- JOSEPH JEAN-MARC ET AL: "High level of stabilized angiostatin mediated by adenovirus delivery does not impair the growth of human neuroblastoma xenografts.", CANCER GENE THERAPY NOV 2003, vol. 10, no. 11, November 2003 (2003-11), pages 859-866, XP002565050, ISSN: 0929-1903
- BOUQUET C ET AL: "Systemic administration of a recombinant adenovirus encoding a HSA-angiostatin kringle 1-3 conjugate inhibits MDA-MB-231 tumor growth and metastasis in a transgenic model of spontaneous eye cancer", MOLECULAR THERAPY, ACADEMIC PRESS, SAN DIEGO, CA, US, vol. 7, no. 2, 1 February 2003 (2003-02-01), pages 174-184, XP002345651, ISSN: 1525-0016

## Description

### Field of the invention

The present disclosure relates to a conjugate comprising angiostatin and its fragments, preferable fragment K1-3002C a sustained-release formulation comprising the conjugate and a method for preparing them. The conjugate of the present disclosure has no toxic side effects, exhibits higher biological activities and is more metabolically stable. The present disclosure also provides a pharmaceutical composition comprising the conjugate or the sustained-release formulation and a kit containing the conjugates. The present disclosure also relates to the use of the conjugate, the sustained-release formulation, the pharmaceutical composition and the kit for preventing, diagnosing, and treating tumors, for preparing antitumor medicaments and for preventing diseases related to neovascularization.

### Background of the invention

Cancer is a common and frequently-occurring disease nowadays, as well as the number one killer threatening human life. Therefore, the research and development of anti-cancer drugs is a hot topic in China and abroad. Currently, the anti-cancer drugs commonly used for clinical treatments are traditional chemotherapy drugs, which can easily lead to resistance and have many toxic side effects during long-term usage. Therefore, their clinical applications have been greatly restricted.

In 1971, Folkman proposed the theory that the growth and migration of tumor cells depend on angiogenesis, the growth of new capillaries from existing blood vessels. Therefore, blocking tumor angiogenesis may inhibit tumor growth and metastasis. Taking tumor vascular endothelial cells as the target of cancer therapy provides a new strategy for cancer treatment, i.e. anti-angiogenesis therapy (Folkman J. N Engl J Med 1971;285: 1182 -1186).

Angiostatin is the endogenous angiogenesis inhibitor extracted by O'Reilly et al. from serum and urine of Lewis lung tumor-bearing mice. It is the N-terminal sequence of plasminogen starting from the 98th amino acids (using the initial Met as the first amino acid) (O'Reilly MS, Holmgren L, Shing Y, et al. Cell, 1994, 79: 315-328), including at least the first four Kringles structures of five, each of which consists of about 80 amino acids (Lerch P G, Riskli E E, Lergier W, et al. Eur J Biochem, 1980, 107: 7-13).

It has been shown that the fragments K1, K2, K3, K5, K1-3, K2-3 and K1-5 of recombinant human plasminogen can effectively inhibit bovine capillary endothelial cell (BCE) proliferation, while K4 almost has no such activity. The inhibitory effect of K1-3 on cell proliferation is significantly stronger than that of the initially-discovered angiostatin K1-4 (Cao Y, Ji RW, Davidson D, et al. J Biol Chem, 1996, 271(46): 29461-29467.). It has also been shown that K1-3 can not only suppress the growth and metastasis of mouse Lewis lung cancer, but also inhibit the growth of a variety of primary tumors such as mouse Lewis lung cancer primary tumor and hemangioendothelioma. The mechanism underlying the activities of angiostatin is that it suppresses the angiogenesis in tumor tissues by inhibiting the growth of vascular endothelial cells. Consequently, the tumor cannot obtain necessary nutrition and oxygen needed for its growth, which results in growth inhibition and starvation. O'Reilly pointed out that angiostatin is a fragment within endogenous plasminogen, which has specific effect on proliferating vesicular endothelial cells. Therefore, it will generally not cause immune response, as well as side effects such as loss of weight, reduced activities, loss of appetite and conditioned pathogen infection under long-term injection (Sim BKL, O'Reilly MS, et al. Cancer Res, 1997, 57: 1329-1334.). At the same time, it will not affect the liver and kidney function (Kirsch M, Strasser J, Allende R, et al. J Biol Chem, 1996, 271(46): 29461-29467). In contrast, most of commercially available chemotherapeutic drugs are targeted on tumor cells that are genetically instable and highly mutative, resulting in a high risk of resistance and thus decrease the efficacy. Until recently, the use of angiostatin in tumor treatment has shown promising results. For the above reasons, angiostatin is expected to become a main anti-cancer drug in 21st century.

Compared to traditional chemical drugs, polypeptide and protein drugs including angiostatin have the advantages of causing low toxicity/side effects or little drug resistance etc, and in order to increase bioavailability and decrease degradation *in vivo*, the protein drugs are usually administrated intravenously. However, the half-life of low molecular weight protein drugs is very short, not only because of the degradation, but also because of the quick elimination via kidney.

It has been shown that in blood, if the hydraulic radius of a protein is larger than that of albumin or if the molecular weight of a protein is greater than 66,000 Dalton, the protein can be reserved stably in circulation. However, protein of lower molecular weight will be quickly eliminated from the blood via glomeruli. As such, in order to maintain the effective treatment concentration of low molecular weight proteins in blood, frequent injection or continuous infusion is required. Although treatment in such a way could achieve the therapeutic effects, it also causes many inconveniences and pains to the patients and increases the cost of treatment. Meanwhile, long term administration of some drugs may cause some side effects, for instance, immunological reactions.

As a protein drug, angiostatin also has the disadvantages of short half-life and high elimination rate *in vivo*. The purpose of the present invention is to improve metabolism characteristics of angiostatin, which would result in higher stability, longer half-life, and even better efficacy *in vivo.*

Using macromolecular polymer to modify proteins is a common method to change and control the dynamic characteristics of drugs such as half-life, immunological characteristics, and toxicological characteristics. The macromolecular polymer employed to modify proteins should have desirable water-solubility and biocompatibility, little or no immunogenicity and so forth. Polyethylene glycol (PEG) is a common protein-modified molecule. It is amphipathic, and can be dissolved not only in water, but also in most of the organic solvents. Since polyethylene glycol is non-toxic, non-immunogenic, and highly soluble in water, it has been approved as a macromolecular polymer for drug preparation by drug administrations in many countries including SFDA of China as well as FDA of the US. Coupling the protein with macromolecular polymers, for example, polyethylene glycol can increase the *in vivo* stability and decrease nonspecific adsorption and antigenicity of the protein. Once the molecular weight of the conjugate reaches a certain value, the eliminating rate by kidney can be reduced effectively, which is an effective measure to prolong the half-life of the protein drug *in vivo* (Frokjaer S. et al. Nat. Rev. Drug Discov. 2005 Apr 4(4): 298-306; Harris JM. et al. Nat. Rev. Drug Discov. 2003 Mar 2(3): 214-21). The amino groups used as the reaction site in the modification of PEG were mainly α-amino group of N-terminus of the protein and ε-amino group on the side chain of the lysine residue. The result of the reaction is the coupling of one protein molecule with one or multiple PEG molecules. The modification of ε-amino group on the side-chain of the lysine residue may result in modified isomers due to the non-specific reaction sites.

Recently, taking advantage of the difference of isoelectric points between α-amino group of N-terminus of the protein and ε-amino group on the side chain of the lysine residue, polyethylene glycol modifying agents have been developed which specifically modify N-terminus of the protein and as a result, it is possible to obtain uniform modification products with modification at the identical site. Another protein site for modification with polyethylene glycol is the mercapto group of the cysteine residue. Generally, the number of the mercapto groups is fewer than that of the amino groups in a protein, and thus modification of the mercapto groups is more specific. Using genetic engineering techniques, now it is possible to introduce a cysteine at any position of a protein to serve as a specific modification site. However, introducing a cysteine as the modification site also has certain limitations because, for those proteins that already contain cysteine residues, this may cause a mispairing of disulfide bonds or make it difficult for such proteins to refold. In addition, the carboxyl group of the protein is also frequently used as a site for modification (Veronese FM et al. Drug Discov. Today. 2005 Nov 1;10(21):1451-8.). Modification techniques with polyethylene glycol have been successfully used in a plurality of protein drugs, including PEG-asparaginase (Graham ML Adv. drug Deliv. Rev. 55, 1293-1302 Avramis Vassilios I. et al. WO9939732 and US6689762), PEG-adenosine deaminase (Levy Y et al. J. Pediatr. 113, 312-317; Davis S et al. ClinExp. Immunol. 46: 649-652; Hershfield MS et al. N Engl J Med 316 : 589-596), PEG-interferons such as PEG-interferon α2a and PEG-interferon α2b etc. (Bailon P et al. C. Bioconjug. Chem. 12, 195-202, Wang YS et al. Adv. Drug Deliv. Rev. 54, 547-570, Meng Xiantai et al. WO2005077421, Van Vlasselaer Peter et al. WO2004076474, Ballon Pascal Sebastlan et al. US2004030101, Karasiewicz Robert et al. EP0593868) etc. The purpose of this lies in obtaining fragments of angiostatin or complex containing angiostatin with prolonged half-life and higher biological activities.

Other representative high molecular modifying agents include glucan, polysucrose, starch, polyalanine, copolymer of ethanedioic acid and the malonic acid, carboxymethyl cellulose, polyvinylpyrrolidone, poly 1,3-dioxolane, copolymer of ethylene and maleic hydrazide, poly sialic acid, cyclodextrin, etc.

Another method of prolonging the half-life of a protein drug is to couple or fuse it with a blood protein or a fragment thereof used as a carrier to increase the molecular weight of the protein drug. For example, Fc fragment of immunoglobulin may be coupled with the target protein to prolong the half-life of the latter. For example, this strategy has been used in the Notch 1 receptor protein (Kitajewsky Jan et al. WO2005111072), erythropoietin (EPO) (Gillies Stephen D et al. WO2005063808), human somatropin (Kim Young Min et al. WO2005047337), etc. The plasma albumin is another commonly-used coupling carrier, which is used in the proteins such as some antibiotics, anti-inflammatory agents, and anti-oxidants (Ezrin Alan M et al. WO0117568, Otagiri Masaki et al. EP1571212), etc.

Sustained release formulation is another approach of prolonging the *in vivo* half-life of a protein drug. The protein drug is placed in a pharmaceutical carrier to allow the protein to be released from the carrier slowly, and thereby a stable *in vivo* drug concentration is maintained. The commonly used sustained release formulations comprise hydrogel, microcapsule, microballoon, micro-osmotic pump, liposome etc. (Peppas NA et al. Eur. J. Pharm. Biopharm. 50, 27-46 (2000); Packhaeuser CB et al. Eur. J. Pharm. Biopharm. 58, 445-455 (2004); Metselaar JM et al. Mini Rev. Med. Chem. 4, 319-329 (2002)). Liposome is an ultramicroscopic particle in the form of a hollow sphere with a bilayer membrane structure. The bilayer membrane is composed of amphipathic molecules, most of which are phosphatides, and forms a hydrophilic inner chamber. The hydrophilic protein drug is encapsulated in the inner chamber of the liposome and thus can be slowly released *in vivo* to maintain the concentration of the protein drug and prolong the half-life. Examples are nerve growth factor (Hou Xinpu et al. CN1616087) and hemoglobin (Farmer Martha C et al. US4911929) etc.

Joseph et al. Cancer Gene Therapy (2003) 10(11), 859-866 and Bouquet et al. Molecular Therapy (2003) 7(2), 174-184 relate to adenoviral delivery of conjugates of the K1-3 fragment with human serum albumin.

### Summary

The present invention provides a conjugate of polyethylene glycol and angiostatin fragment K1-3, wherein the conjugate exhibits prolonged *in vivo* half-life and enhanced biological activity as compared to unmodified angiostatin fragment K1-3, and wherein the conjugate is formed by coupling one angiostatin fragment K1-3 with one polyethylene glycol molecule at the N-terminal α-amino group of the angiostatin fragment K1-3, and said angiostatin fragment K1-3 consists of a sequence as shown in SEQ ID NO:1 or a sequence having at least 80% amino acid sequence homology to SEQ ID NO:1, and wherein said polyethylene glycol molecule has a molecular weight of 5,000 to 40,000 Daltons.

Further aspects of the invention are defined in the claims.

"K1", "K2", "K3", "K5", "K1-2", "K1-3", "K1-5", "K2-3", "K2-5", "K3-5" and "kringles1-3" mentioned in this disclosure, unless indicated otherwise, are fragments of plasminogen, and the sequence of human plasminogen is shown in SEQ ID NO:7(refer to U.S. Patent NO:7, 157,556 and WO 02/26782 A2). "K1-3" here is the abbreviation of "Kringles1-3". Unless otherwise indicated, PEG is polyethylene glycol, preferably monomethyl polyethylene glycol (mPEG).

Another aspect of the disclosure is that, the inventor surprisingly discovered that a conjugate formed by modifying agents and angiostatin or its fragments has prolonged *in vivo* half life as compared to the unmodified angiostatin or its fragments. The agents used for modification are selected from macromolecular polymers, protein molecules or its fragments, peptides, small molecules or chemicals of other forms.

The conjugate not only maintains its original activity of inhibiting angiogenesis, but also exhibits higher biological activity and longer *in vivo* half life. Moreover, it does not exert toxic side effects on cells and animals, and even for long time use it does not result in side effects such as resistance. In addition, Henkin et al. reports that the conjugate formed by PEG and K5 maintains the original biological activity of K5, and its half life increases to 5.53h (WO 02/26782 A2). Surprisingly, the half-life of the conjugate formed by PEG and K1-3 mentioned herein increases to 81.5h, much higher than that of the conjugate formed by PEG and K5 (refer to the example 4). For clinical application purpose, a longer delivery interval or a smaller dose of the drug is of great importance to patients, whether out of economic or mental health consideration.

In a preferred embodiment, the angiostatin of the present invention is of human, mice or other mammal origin. Preferably, the angiostatin is human angiostatin.

In another preferred embodiment, the angiostatin of the present invention is recombined angiostatin. More preferably, the angiostatin is human recombined angiostatin.

Disclosed herein are fragments of angiostatin selected from angiostatin K1, K2, K3, K5, K1-2, K1-3, K1-5, K2-3, K2-5 and K3-5.

Angiostatin K1 may have the sequence shown in SEQ ID NO:3, angiostatin K2 the sequence shown in SEQ ID NO:4 or angiostatin K3 the sequence shown in SEQ ID NO:5. The fragments of angiostatin mentioned here include angiostatin K1-3 having the sequence shown in SEQ ID NO:1. When the angiostatin mentioned is recombined angiostatin K1-3 expressed by *E.coli,* it has the sequence shown in SEQ ID NO:2, and the N-terminal Met is randomly deleted when expressed in *E.coli.*

The angiostatin or its fragments of the present disclosure also includes the biologically active mutant, derivative, isomer or the combination thereof. In one case, the derivative of angiostatin or its fragment is formed by adding a peptide of 1-15 amino acids to the N or C terminal of the angiostatin or its fragments. In a further case, the derivative of angiostatin or its fragments is formed by adding a 7-amino-acid peptide MHHHHHH containing a His-tag to the N-terminal of human angiostatin. This derivative has the sequence shown in SEQ ID NO:6 and its N-terminal Met are randomly deleted when expressed in *E. coli.* In another case, the derivative of angiostatin or its fragments is formed by adding the peptide MGGSHHHHH or MGGSHHHHHH to the C terminal of angiostatin, and its N-terminal Met is randomly deleted when expressed in *E.coli*

In one of the aspects of this disclosure, the modifying agents in the conjugate of the present invention are linked to angiostatin or its fragments by covalent bonds. In another aspect of this disclosure, the modifying agents in the conjugate are linked to angiostatin or its fragments by non-covalent bonds. Preferably, the modifying agents are linked to angiostatin or its fragments through chemical coupling or fusion and expression. The macromolecular polymers as mentioned above can be biologically active or not. The suitable macromolecular polymers include, but not limited to, polyenol compounds, polyether compounds, polyvinylpyrrolidone, poly amino acids, copolymer of divinyl ether and maleic anhydride, N-(2-Hydroxypropyl)-methacrylamide, dextran and its derivatives like dextran sulfate, cellulose and its derivatives (including methyl-cellulose and carboxymethyl cellulose), amylum and its derivatives, poly-sucrose, polyethoxylpolyol, heparin or its fragment, polyalkylene glycol and its derivatives, copolymers of polyalkylene glycol and its derivatives, poly(vinyl ethyl ether), a,p-poly((2-hydroxyethyl)-DL-aspartamide, polycarboxylates, poly oxyethylene-oxymethylenes, polyacryloyl morpholines, copolymer of amino compounds and oxyalkylene, poly hyaluronic acid, polyoxiranes, copolymer of oxalic acid and malonic acid , Poly 1,3 - methylene glycol ether, copolymer of ethylene and maleic hydrazide, poly sialic acid, cyclodextrin and etc.

The polyethers as mentioned above include, but are not limited to, poly alkylene glycol (HO((CH2)xO)nH), polypropylene glycol, polyoxyethylene (HO((CH₂)₂O)ₙH), polyvinyl alcohol ((CH₂CHOH)ₙ) and derivatives thereof. The polyethylene alcohols as mentioned above include, but are not limited to, polyethylene glycol (monomethyl polyethylene glycol, hydroxypolyethylene glycol), polyvinyl alcohol, polypropylene alcohol, polybutene alcohol and their derivatives. In one case, the modifying agent is polyethylene glycol. In some cases, polyethylene glycol is monomethyl polyethylene glycol.

The polyethylene glycol (PEG) of present invention is either linear or branched.

The molecular weight of PEG used herein is between 5,000 to 40,000 Daltons. Most preferably, the PEG used herein is 20 kDa monomethyl polyethylene glycol.

In one aspect of the present disclosure, the polyethylene glycol (PEG)-modified angiostatin is characterized in that one angiostatin molecule is covalently coupled to one or more PEG molecules. Preferably, the modification is site specific and will not influence the bioactivity of angiostatin or its fragment. This modified product maintains the antiangiogenic activity and shows higher metabolic stability and longer half-life in blood than the unmodified angiostatin, and therefore it can be used as an antiangiogenic or anti-tumor conjugate. Preferably, the coupling site of angiostatin may be at the N-terminal α-amino group, the ε-amino group on the side chain of a lysine residue, the mercapto group of on the side chain of a cysteine residue, the carboxyl group on the side chain of an aspartate residue, the carboxyl group on the side chain of a glutamate residue, or any combination thereof. More preferably, the coupling site of angiostatin or its fragment used herein is selected from the N-terminal α-amino group of said angiostatin fragment K1-3 or the ε-amino group on the side chain of a lysine residue corresponding to the 2nd, 7th, 15th, 17th, 24th, 69th, 94th, 97th, 121 st, 125th, 128th, 129th, 150th, 175th, 215th, 228th, 246th lysine residue as indicated in SEQ ID NO:1 or any combination thereof. More preferably, one angiostatin molecule or its fragment is coupled with one polyethylene glycol molecule at the N-terminal α-amino group of said angiostatin molecule. More preferably, in the conjugate, one recombinant angiostatin fragment K1-3 shown as SEQ ID NO:2 is coupled with one polyethylene glycol molecule. The coupling site is the N-terminal α-amino group of the angiostatin fragment K1-3. Most preferably, in the conjugate, one recombinant angiostatin fragment K1-3 shown as SEQ ID NO:2 is coupled with one 20 kDa monomethyl polyethylene glycol. The coupling site is the N-terminal α-amino group of the angiostatin fragment K1-3. In a preferred case, PEG is used to modify the N-terminal α-amino group of K1-3 and the product obtained has inhibitory activities on the proliferation, migration of vascular endothelial cells, the proliferation of tumor cells in mice, and the biological activity of the tumor growth in mice.

In one case disclosed herein, the conjugate is one angiostatin molecule covalently coupled to one or more polyethylene glycol molecules at the mercapto groups on the side chains of additional cysteine residues of said angiostatin molecule, wherein the additional cysteine residues are introduced by adding cysteines or peptide chains containing cysteines to the N-terminus, C-terminus, or the internal region of said angiostatin molecule.

Poly amino acids include, but not limited to, polymers of one type of amino acid, copolymers of two or more types of amino acids, for example, polyalanine.

According to the present disclosure, preferred protein molecule used as a modifying carrier is a protein naturally occurring or a fragment thereof, which includes, but not limited to, thyroxine-binding protein, transthyretin, transferrin, fibrinogen, immunoglobulin, albumin and fragments thereof. Preferably carrier proteins are human proteins. A fragment of a protein as mentioned above refers to a part of the protein that keeps the function of a carrier protein. In one case, angiostatin or its fragment is coupled to albumin, characterized in that one angiostatin molecule or its fragment is linked to one or more albumin molecules. The conjugate may be obtained via chemical modification, fusion expression, or other methods. Preferably, the albumin is human serum albumin or its fragment. In another case, the conjugate is one angiostatin molecule or its fragment linked to Fc fragment of human immunoglobulin IgG. It is characterized in that one angiostatin molecule is covalently coupled to one or more Fc fragment of human immunoglobulin IgG. The conjugate may be obtained either via chemical modification, or via fusion expression, or via other methods. Preferably, the Fc fragment is a fragment of the Fc region of human immunoglobulin IgG.

The modifying agent herein also includes a small molecule or a small peptide or other compound. In one case, the conjugate is an angiostatin molecule or its fragment modified by a small molecule or a small peptide or other compound. It is characterized in that the product has the activity to react with or bind to other molecules or component of the body. The reactivity mentioned can be of a reactively active group, capable of reacting with amino, carboxyl or mercapto group to form a covalent bond. In another specific case, the active group of the product is maleimide, which is capable of reacting with a mercapto group of a blood component, such as albumin. In another case, the conjugate is the product of the modification of an angiostatin molecule or its fragment by glycosylation, phosphorylation or acylation. The modification sites may be amino acid residues originally contained the protein or amino acid residues generated via mutation. Although the molecular weight of the conjugate is not very large, the half-life of the conjugate becomes longer since the modification changes the characteristics of K1-3.

Angiostatin or its fragment conjugates is directly or indirectly linked to the modifying agent via a covalent bond. Direct linking means that an amino acid of angiostatin or its fragment is directly linked to an amino acid of the carrier protein, via peptide bond or a disulfide bridge. Indirect linking means that angiostatin or its fragment conjugates is linked to the carrier protein via one or more chemical groups. In another aspect of the present disclosure, the conjugate comprising angiostatin or its fragment is a conjugate formed via non-covalent interaction between angiostatin or its fragment and a modifying agent such as protein and small molecule.

Another aspect relates to a sustained-release formulation comprising angiostatin or its fragment, preferably fragment K1-3, or an angiostatin containing conjugate and a bio-compatible substance. The sustained-release formulation may be in a form selected from the group consisting of microcapsule, hydrogel, microsphere, micro-osmotic pump or liposome.

The present disclosure also provides a pharmaceutical composition comprising a conjugate or sustained-release formulation of the present invention and a pharmaceutically acceptable carrier, wherein the conjugate is formed by a modifying agent and angiostatin or its fragment, preferably angiostatin fragment K1-3. The pharmaceutically acceptable carriers include carriers, excipients, or stabilizers, which are nontoxic to the cells or mammals to be contacted at selected doses and concentrations. A commonly used physiologically acceptable carrier is an aqueous pH buffer solution. Examples of physiologically acceptable carriers include solutions such as phosphate buffer solution, citrate buffer solution and other organic acid buffer solution; anti-oxidants including ascorbic acid; polypeptides with low molecular weight (no more than 10 residues); proteins such as serum albumin, glutin or immunoglobulin; hydrophilic polymers such as polyvinylpyrrolidone; amino acids such as glycine, glutamine, asparagine, arginine or lysine; monosaccharide, disaccharide and other carbohydrates including glucose, mannose or dextrin; chelating agents such as EDTA; sugar alcohol such as mannitol or sorbitol; salt-forming counter ion such as sodium ion; and/or nonionic surfactant such as TWEEN®, PEG and PLURONICS®. Preferably, the excipients are sterilized and usually free of undesired substance. These compositions can be sterilized by routine sterilizing techniques.

The present disclosure also provides kits comprising the conjugate, composition or sustained-release formulation and instruction for the use thereof. The complex is composed of a modifying agent and angiostatin or its fragment, preferably PEGylated angiostatin fragment K1-3.

The present disclosure also relates to a method of preparing the conjugate, which is formed by a modifying agent and angiostatin or its fragment, preferably fragment K1-3. Specifically, the present disclosure provides a method of preparing a PEGylated angiostatin or the fragment thereof comprising mixing the activated PEG with angiostatin or its fragment, allowing the reaction under proper conditions including solution, temperature, pH, and reaction molar ratio, and purifying the coupled product by using cation-exchange column or molecular sieve. Preferably, the pH of this reaction is within 3 to 10. Preferably pH 5 to 7, the molar ratio of PEG and angiostatin is 1:1 to 10:1.

The present disclosure also provides the use of the aforementioned angiostatin-containing conjugate, formed by a modifying agent and angiostatin or its fragment preferably fragment K1-3, the sustained-release formulation, the pharmaceutical composition or the kit in the prevention, diagnosis and treatment of tumor. The tumors that are suitable to be treated with the method of the invention include, but not limited to, lung cancer, neuroendocrine tumor, colon cancer, bone cancer, liver cancer, gastric cancer, pancreatic cancer, oral cancer, breast cancer, prostate cancer, lymphoma, esophagus cancer, nasopharyngeal carcinoma, cervical cancer, sarcoma, renal carcinoma, biliary cancer, malignant melanoma, et al. The present disclosure also provides the use of the aforementioned conjugate, sustained-release formulation or pharmaceutical composition comprising the angiostatin or its fragment preferably K1-3, in the preparation of a medicament for the treatment of aforementioned tumors. The administration route is selected from the group consisting of intravenous injection, intravenous infusion, subcutaneous injection, intramuscular injection, intraperitoneal injection, percutaneous absorption, subcutaneous embedment, liver arterial injection, oral administration, nasal administration, oral mucosa administration, ocular administration, rectal administration, vaginal delivery, or other clinically acceptable routes. The administration time is every day to every 21 days, preferably every day to every week.

The present disclosure also provides the use of the aforementioned conjugate, sustained-release formulation, pharmaceutical composition, or kit for the prevention, diagnosis, or treatment of a non-cancerous disease characterized by, but not limited to, tissue or organ pathological changes caused by abnormal neovascularization, or in preparation of a medicament for the treatment of such a disease, wherein the conjugate is formed by a modifying agent and angiostatin or its fragment, preferably said conjugate is angiostatin fragment K1-3 modified by PEG. According to the present disclosure, the administration route is selected from the group consisting of intravenous injection, intravenous infusion, subcutaneous injection, intramuscular injection, intraperitoneal injection, percutaneous absorption, subcutaneous embedment, liver arterial injection, oral administration, nasal administration, oral mucosa administration, ocular administration, rectal administration, vaginal delivery, or other clinically acceptable routes. The administration frequency is selected from daily to every 21 days, preferably daily to weekly.

The present disclosure also provides a method for prolonging the half-life of angiostatin or its fragments, preferably fragment K1-3, comprising the step of forming a conjugate between angiostatin or its fragments and a modifying agent capable of prolonging in vivo half-life of angiostatin and optionally the step of preparing a sustained-release formulation containing the aforementioned conjugate and biocompatible substances. Our experimental data show that, a product disclosed herein, i.e., a conjugate formed by coupling PEG to the N-terminus of K1-3, has the activity of inhibiting the migration of endothelial cell and tumor growth in mice. This conjugate showed significantly higher activity as compared with non-modified K1-3. Furthermore, pharmacokinetic study revealed that the in vivo metabolism of this modified product was effectively slowed down, and its in vivo half-life was prolonged.

Unless otherwise specified, the angiostatin or its fragments as used herein can be a wild-type angiostatin, i.e., the naturally occurring form; or a biologically active mutant, fragment, isomer, or derivative thereof, or their combinations. Angiostatin or its fragments can be obtained upon but not limited to expression in animal cells, or can be obtained by fermentation and further purification from yeasts or E. coli. The amino acid sequence of the wild-type human angiostatin fragment K1-3 expressed in animal cells or yeasts is shown in SEQ ID NO.1. The recombinant human angiostatin or its fragments expressed in animal cells or yeasts may either have an amino acid sequence as shown in SEQ ID NO.1, or have the sequence of SEQ ID NO.1 with no more than 10 amino acids added to or deleted from its N-terminus. The amino acid sequence of the recombinant human K1-3 expressed in E. coli is shown in SEQ ID NO.2, but sometimes the N-terminal Met will be deleted after expression.

A mutant of K1-3 refers to a K1-3 molecule obtained by introducing amino acid replacement, deletion, or addition. The fragment of K1-3 refers to a part of the sequence of SEQ ID NO.1 or SEQ ID NO.2. The fragment may be obtained through, among others, enzymatic cleavage, genetic engineering expression, or polypeptide synthesis. Preferably, the mutant have a 60% amino acid sequence homology to SEQ ID NO:1, or 70% amino acid sequence homology, more desirably 80% or even 90% amino acid sequence homology, especially 95% amino acid sequence homology. A fragment refers to a part of the sequence of SEQ ID NO:1. For instance, the fragment with the sequence of SEQ ID NO:3, SEQ ID NO:4, or SEQ ID NO:5 was reported to have the activity to inhibit endothelial cell proliferation (Cattaneo MG et al. THE JOURNAL OF BIOLOGICAL CHEMISTRY. Vol. 271, No. 46, Issue of November 15, pp. 29461 -29467, 1996). An isomer of K1-3 refers to a molecule with the same amino acid sequence or chemical formula as SEQ ID NO:1 but with different conformations, including difference in the secondary or tertiary protein structure, or changed optical activity in regional amino acids. The isomer may be a naturally occurring mutant or obtained via artificial design. A derivative of K1-3 refers to a product generated by modification of the SEQ ID NO:1. Modification refers to covalently attaching one or more small molecules, such as phosphoric acid molecules or carbohydrate molecules, or oligopeptides with less than 30 amino acids, to the protein at any amino acid of the protein. The combination of the active mutants, fragments, isomers or derivatives of K1-3 means that the product may simultaneously have two or more of the modifications mentioned above, for example without limitation, a mutant of a fragment or a modified mutant, et al.

### Brief description of the drawings

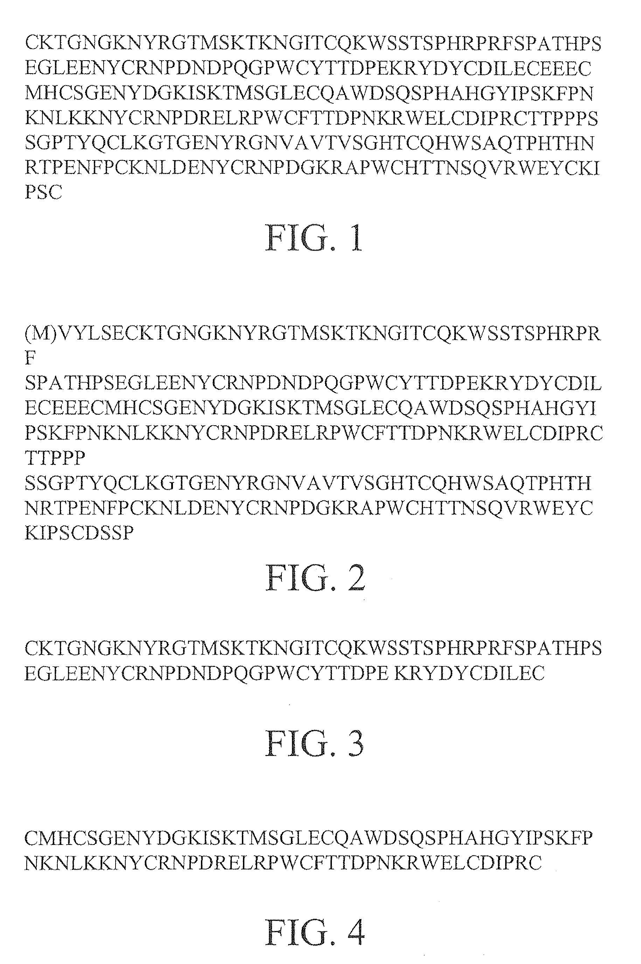

FIG. 1 depicts the amino acid sequence of angiostatin fragments K1-3 (SEQ ID NO:1)
FIG. 2 depicts the amino acid sequence of angiostatin fragments K1-3 (SEQ ID NO:2) expressed in a strain of *E.coli.* The Met residue in the N-terminal may sometimes be deleted after expression.
FIG. 3 depicts the amino acid sequence of angiostatin k 1 (SEQ ID NO:3).
FIG. 4 depicts the amino acid sequence of angiostatin k 2 (SEQ ID NO:4).
FIG. 5 depicts the amino acid sequence of angiostatin k 3 (SEQ ID NO:5).
FIG. 6 depicts a biologically active fragment of K1-3 with additional amino acids at the N-terminus (SEQ ID NO: 6). N-terminal Met may sometimes be partially deleted after expression.
FIG. 7 shows the coupling of PEG with the N-terminus of K1-3 having the sequence of SEQ ID NO:2 at 4□ for 10h. The post-modification solutions are subjected to SDS-PAGE detection. Lane 1, protein marker; lane 2, K1-3; lanes 3~8, K1-3 modified with PEG in a 1:5 molar ratio (K1-3:PEG) at pH 5.0, 5.5, 6.0, 6.5, 7.0, 8.0 respectively; and lanes 9-12: K1-3 modified with 20kDa PEG in a molar ratio (K1-3:PEG) of 1:1, 1:2, 1:5 and 1:10 respectively at pH 5.5.
FIG. 8 shows the coupling of PEG with the N-terminus of K1-3 having the sequence of SEQ ID NO:2 at room temperature for 10h. The post-modification solutions are subjected to SDS-PAGE detection. Lane 1, protein marker; lane 2, K1-3; lane 3~8, K1-3 modified with PEG in a 1:5 molar ratio at the pH of 5.0, 5.5, 6.0, 6.5, 7.0, 8.0 respectively; and lane 9~12, K1-3 modified with PEG in a molar ratio (K1-3:PEG) of 1:1, 1:2, 1:5 and 1:10 respectively at pH 5.5.
FIG. 9 shows the coupling of PEG to the N-terminus of K1-3 having the sequence of SEQ ID NO:2. The pre- and post-modification solutions are subjected to SDS-PAGE detection. Lanel, protein marker; lane 2, K1-3; and lane 3, K1-3 modified with PEG.
FIG. 10 shows the cation-exchange chromatography SP purification of K1-3 having the sequence of SEQ ID NO:2 modified at the N-terminus with PEG. The protein are bound to the cation-exchange column SP, eluted with solution of a sodium chloride gradient, and subjected to reduced SDS-PAGE detection. Lane 1, protein marker; lane 2, before purification; lane 3, flow through; lane 4-13: collected elution fractions.
FIG. 11 shows the result of the *in vivo* metabolism experiment of the coupling product of PEG to the N-terminus of K1-3. K1-3: K1-3 having the sequence SEQ ID NO:2; PEG-K1-3: K1-3 having the sequence of SEQ ID NO:2 which is modified with PEG.
FIG. 12 shows the activity of the coupling product of PEG to the N-terminus of K1-3 of inhibiting the migration of human umbilical vein endothelium cells (HUVECs). K1-3: K1-3 having the sequence SEQ ID NO:2; PEG-K1-3: K1-3 having the sequence of SEQ ID NO:2 which is modified with PEG.
FIG. 13 shows the activity of the coupling product of PEG to the N-terminus of K1-3 of inhibiting the self-regeneration of HUVECs, K1-3: K1-3 having the sequence SEQ ID NO:2; PEG-K1-3: K1-3 having the sequence of SEQ ID NO:2 which is modified with PEG.
FIG. 14 shows the activity of the coupling product of PEG to the N-terminus of K1-3 of inhibiting the metastasis of HUVECs. K1-3: K1-3 having the sequence SEQ ID NO:2; PEG-K1-3: K1-3 having the sequence of SEQ ID NO:2 which is modified with PEG.
FIG. 15 shows the activity of the coupling product of PEG to the N-terminus of K1-3 of inhibiting the metastasis of human dermal microvascular endothelial cells (HMECs). K1-3: K1-3 having the sequence SEQ ID NO:2; PEG-K1-3: K1-3 having the sequence of SEQ ID NO:2 which is modified with PEG.
FIG. 16 shows the activity of the coupling product of PEG to the N-terminus of K1-3 of inhibiting the metastasis of tumor cells (Hela). K1-3: K1-3 having the sequence SEQ ID NO:2; PEG-K1-3: K1-3 having the sequence of SEQ ID NO:2 which is modified with PEG.

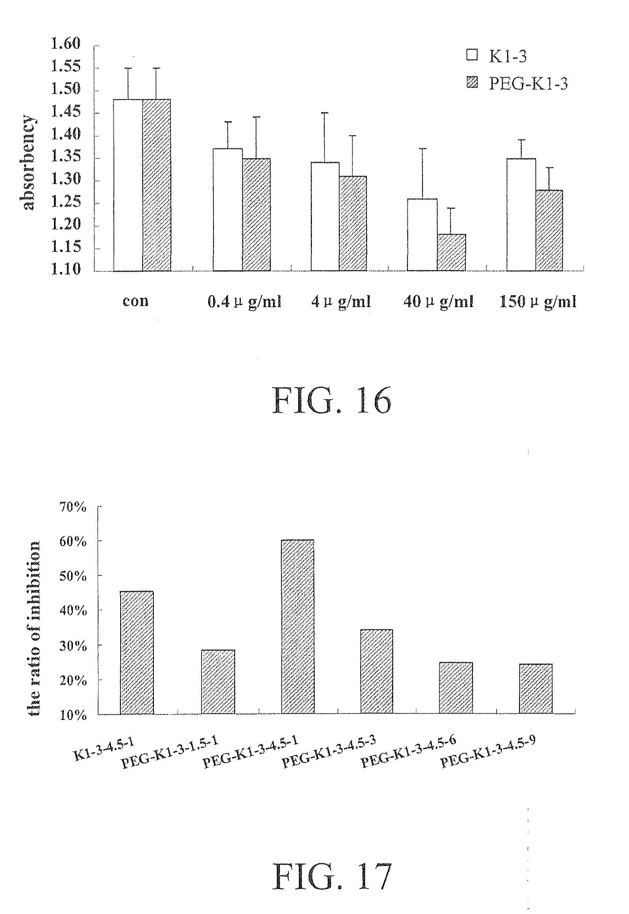

FIG. 17 shows the activity of the coupling product of PEG to the N-terminus of K1-3 of inhibiting mouse melanoma cells (B16). K1-3-4.5-1: K1-3 having the sequence of SEQ ID NO:2, 4.5mg/kg body weight, administered daily; PEG-K1-3-1.5-1: K1-3 having the sequence of SEQ ID NO:2 which is modified with PEG, 1.5mg/kg body weight, administered daily; PEG- K1-3-4.5-1 K1-3 having the sequence of SEQ ID NO:2 which is modified with PEG, 4.5mg/kg body weight, administered daily; PEG- K1-3-4.5-3 K1-3 having the sequence of SEQ ID NO:2 which is modified with PEG, 4.5mg/kg body weight, administered every 3 days; PEG- K1-3-4.5-3 K1-6 comprising the sequence of SEQ ID NO:2 which is modified with PEG, 4.5mg/kg body weight, administered every 6 days; PEG- K1-3-4.5-3 K1-9 comprising the sequence of SEQ ID NO:2 which is modified with PEG, 4.5mg/kg body weight, administered every 9 days.

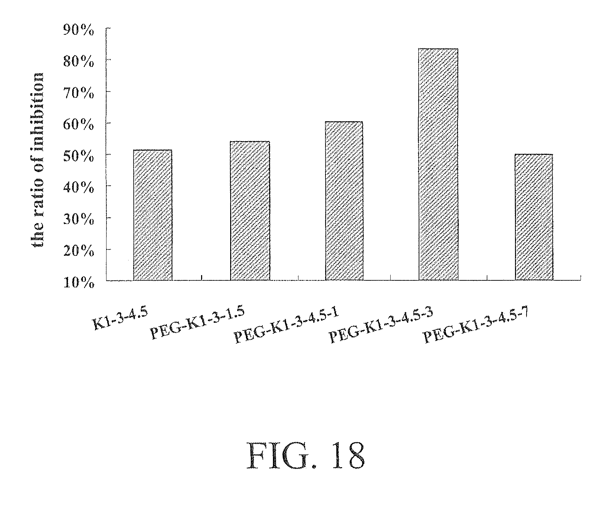

FIG. 18 shows the activity of the coupling product of PEG to the N-terminus of K1-3 of inhibiting mouse liver cancer cells (H22). K1-3-4.5-1: K1-3 having the sequence of SEQ ID NO:2, 4.5mg/kg body weight, administered daily; PEG-K1-3-1.5-1: K1-3 having the sequence of SEQ ID NO:2 which is modified with PEG, 1.5mg/kg body weight, administered daily; PEG- K1-3-4.5-1 K1-3 having the sequence of SEQ ID NO:2 which is modified with PEG, 4.5mg/kg body weight, administered daily; PEG- K1-3-4.5-3 K1-3 having the sequence of SEQ ID NO:2 which is modified with PEG, 4.5mg/kg body weight, administered every 3 days; PEG- K1-3-4.5-3 K1-7 comprising the sequence of SEQ ID NO:2 which is modified with PEG, 4.5mg/kg body weight, administered every week.

### Examples

To further demonstrate the present invention, the following examples are provided. These examples are provided in order to set illustration but not specific limitation to the present invention. , which is defined in the claims.

Unless otherwise specified, the PEG as used herein is 082M0P01 MPEG-BUTYRALD-20K, NETKTAR™, Lot#: PT-03G-05.

### Example 1: Coupling of PEG to the N-terminus of K1-3 under different conditions.

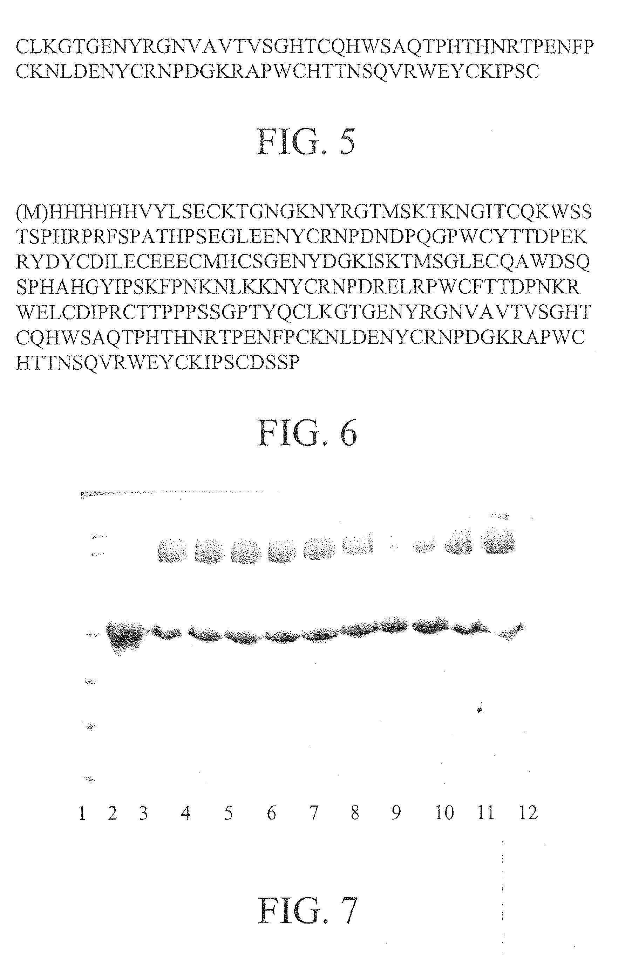

The K1-3 (Protgen Ltd., China) used in this example has the sequence of SEQ ID NO.2. Specifically, the K1-3 was dialyzed into 30 mM sodium acetate solution (Sino Pharm Chemical Reagent Co., Ltd, China), pH 5.5. Protein concentration was determined by measuring absorbance at 280 nm using a UV spectrophotometer (8453, Agilent Technologies), and then was adjusted to 1 mg/ml. PEG which specifically modifies the N-terminus of protein was used to couple with K1-3 covalently. In one experiment, 20 kDa PEG (solid) was added to the K1-3 containing solution obtained after dialysis (containing 10 mg protein) at molar ratios (PEG:K1-3) of 1:1, 2:1, 5:1, 10:1, and the mixed solution was stirred at room temperature until PEG solid dissolved completely. CH₃BNNa (Sigma) was added as reductant to reach a final concentration of 20 mM, and finally the pH value of the solution was adjusted to 5.5. In another experiment, 20 kDa PEG (solid) was added to the K1-3 containing solution obtained after dialysis (containing 10 mg protein) at a molar ratio (PEG:K1-3) of 5:1, and the mixed solution was stirred at room temperature until PEG solid dissolved completely. CH₃BNNa (Sigma) was added as reductant to reach a final concentration of 20 mM, and finally the pH value of the solution was adjusted to 5.0, 5.5, 6.0, 6.5, 7.0, 8.0. After resting at 4°C for different time courses, the following result was observed: one PEG molecule was covalently linked to one K1-3 molecule at N-terminal α-amino group of K1-3. Only a small amount of K1-3 was non-specifically modified at multiple sites. The optimal condition for the interaction between PEG and K1-3 is thereby determined. Electrophoresis analysis results of the solutions before and after the interaction are shown in Figures 7-8.

### Example 2: Coupling of PEG to the N-terminus of K1-3.

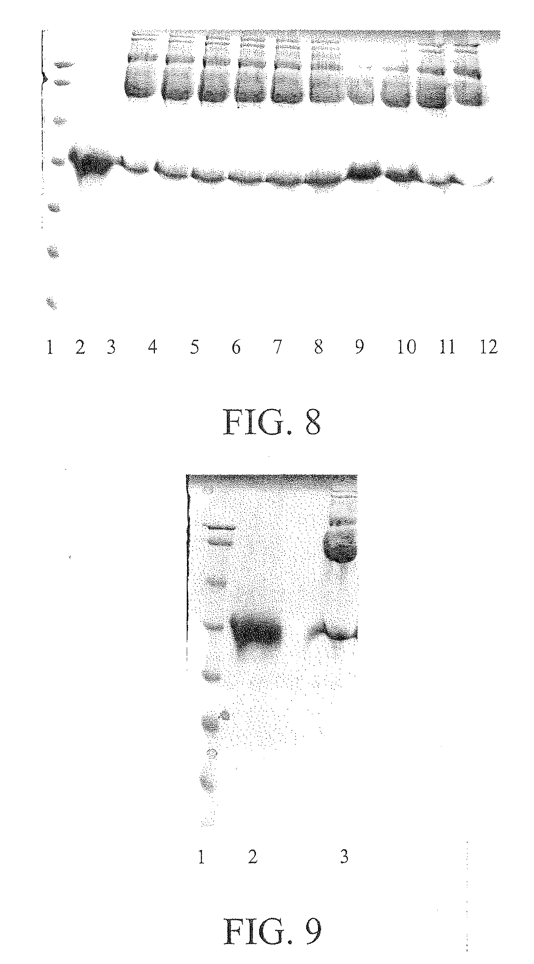

The K1-3 used in this example has the sequence of SEQ ID NO.2. Specifically, the K1-3 was dialyzed into 30 mM sodium acetate solution, pH 5.5. Protein concentration was determined by measuring absorbance at 280 nm using a UV spectrophotometer, and then was adjusted to 1 mg/ml. PEG which specifically modifies the N-terminus of protein was used for covalently coupling with the K1-3. Specifically, 100 mg 20 kDa PEG (solid) was added to 20 ml solution obtained after dialysis (containing 10 mg protein) at a molar ratio (PEG:K1-3) of 5:1, and the mixed solution was stirred at room temperature until PEG solid dissolved completely. CH₃BNNa (Sigma) was added as reductant to reach a final concentration of 20 mM, and finally the pH value of the solution was adjusted to 5.5. After resting at room temperature for 6 hours, more than 80% of K1-3 was modified with PEG at a single site, which means one PEG molecule was covalently linked to one K1-3 molecule at N-terminal α-amino group of K1-3. Only a small amount of K1-3 was non-specifically modified at multiple sites. The reaction solution can be purified directly through chromatography using a column. Electrophoresis analysis results of the solutions before and after the reaction are shown in Figure 9.

### Example 3: Purification of K1-3 modified with 20 kDa PEG at N-terminus through cation-exchange SP column

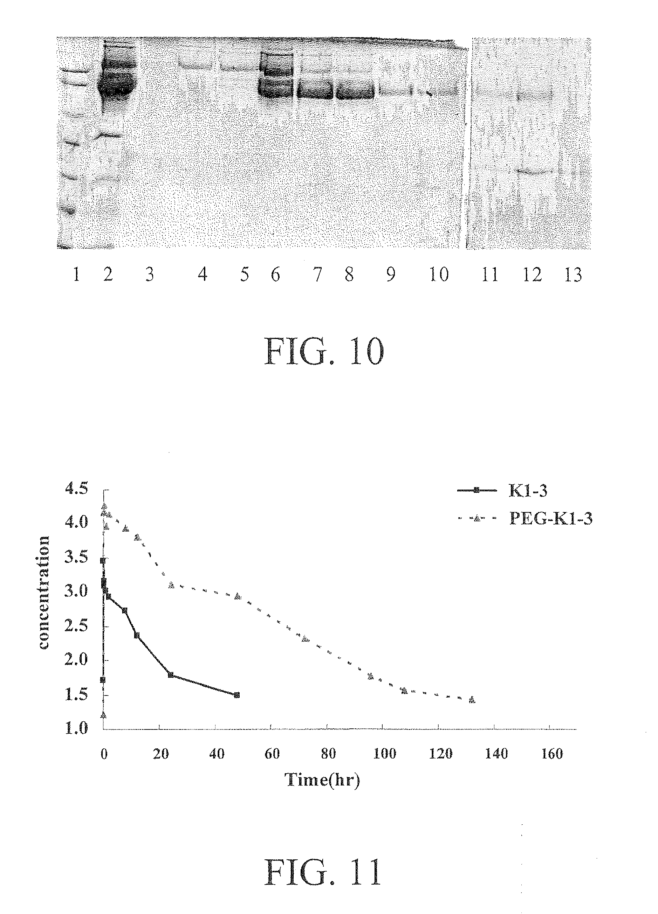

The K1-3 used in this example has the sequence of SEQ ID NO.2. Specifically, K1-3 modified with 20 kDa PEG was purified through SP chromatography column (Amersham). The pH value of the mixed solution obtained after the coupling reaction was adjusted to 5.0. The sample was loaded onto the column pre-equilibrated by a buffer containing 20 mM sodium acetate, pH 5.0. Then gradient elution was performed with buffer containing 20 mM sodium acetate, 1 M NaCl, pH 5.0. The peak of PEG which did not react with K1-3 appeared during penetration and washing since PEG is extremely low charged. The elution peaks appeared in the following order: multi-modified K1-3, mono-modified K1-3, and non-modified K1-3. Different fractions can be collected according to absorbance at 280 nm. The result of purification is shown in Figure 10.

### Example 4: Prolonged efficacy of K1-3 with PEG coupled to its N-terminus in the blood.

The K1-3 used in this example has the sequence of SEQ ID NO.2; The efficacy of tK1-3 with PEG coupled to its N-terminus was evaluated by measuring the *in vivo* half-life of K1-3 with or without PEG modification in Wistar rat (Vitalriver Experimental Animal Center), respectively. Two healthy Wistar rat (about 200 g) were injected intraveniously with K1-3 and K1-3 modified with PEG, respectively, at a dose of 4.5 mg/kg body weight. Blood samples were taken from eye sockets of the rats at 0, 5, 10, 30 minutes, 1, 2, 4, 8, 16, 24, 36, 48, 72, 96, 120 and 144 hours. Plasma was then collected and the concentration of K1-3 with or without PEG modification was measured by sandwich ELISA, respectively. As revealed by the pharmacokinetic analysis, upon modification with PEG, the half-life of K1-3 was increased from 12.2 hours to 81.5 hours in rats. These results demonstrate that coupling of high molecular weight PEG to K1-3 can effectively increase the *in vivo* half-life of K1-3 and result in prolonged efficacy. Results are indicated in Figure 11.

### Example 5: The activity of K1-3 with PEG coupled to its N-tenninus of inhibiting the migration of Human Umbilical Vein Endothelial Cell (HUVEC).

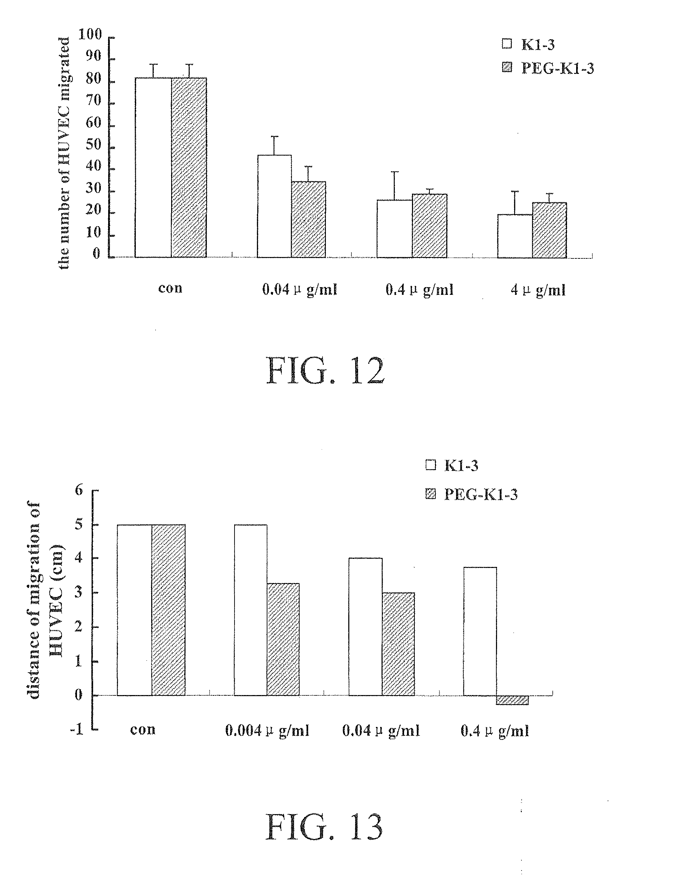

The K1-3 used in this example has the sequence of SEQ ID NO.2. HUVECs (Protgen, Beijing) were cultured in the M199 medium (Hyclone) containing 20% fetal bovine serum (Hyclone) to exponential phase. After starved for 12 hours, cells were added into trans-well inserts (Millipore, USA) at the concentration of 10,000 cells per well for measurement of cell migration. Mediums for migration assay included M199 medium with 5% serum, For the treatment groups, K1-3 with or without modification were added to the cells at 0.004, 0.04, 0.4 and 4 µg/ml. After culturing for 8 hours at 37°C, the cells in the trans-well inserts were fixed with 1% glutaraldehyde (Beijing chemical plant, China). The upper layer of cells on the trans-well membranes, which did not migrate, were removed, and the lower layer of cells were stained with hematoxylin(Sino Pharm Chemical Reagent Co., Ltd, China). The migrated cell number was counted under microscope (Olympus). Cells in three different fields in one plate treatment were counted and then the inhibition rate was calculated. At the concentration of 0.04-4µg/ml, the inhibitory effect of cell migration improves with the increase of protein concentration. The results revealed that the inhibition rates on cells migration were 43%, 68% and 76% for K1-3 with the sequence of SEQ ID NO.2, while 58%, 65%, 69% for modified K1-3. The above results demonstrate that K1-3 modified with PEG will maintain its biological activity. The experimental results are shown in Figure 12.

### Example 6: The activity of K1-3 with PEG coupled to its N-terminus of inhibiting wound healing of Human Umbilical Vein Endothelial Cell (HUVEC).

The K1-3 used in this example has the sequence of SEQ ID NO.2. 2×10⁵ /ml HUVEC were inoculated in 12-well plates (Corning) and cultured in the M199 medium (Hyclone) containing 20% fetal bovine serum (Hyclone) to exponential phase. After starved for 12 hours, crossed wounds were created in each well. Suspending cells were washed away. Experimental groups were as follows: negative control (PBS), positive control (K1-3 at the concentration of 0.04, 0.4 and 4 µg/ml) and treatment group (K1-3 with PEG at the concentration of 0.04, 0.4 and 4 µg/ml). After culturing for 16 hours at 37°C, the amounts of migrated cells were detected. At the concentration of 0.04-4µg/ml, the inhibitory effect of cell migration was improved with the increase of protein concentration. The results revealed that the inhibition rates on cells migration were 0, 20% and 25% for K1-3, while 35%, 40% and 105% for modified K1-3. The above results demonstrate that the modification of K1-3 with PEG will not only maintain but also enhance its biological activity. 4 µg/ml K1-3 modified with PEG resulted in some cell crimple and death. The experimental results are shown in Figure 13.

### Example 7: The activity of K1-3 with PEG coupled to its N-terminus of inhibiting proliferation of Human Umbilical Vein Endothelial Cells (HUVECs).

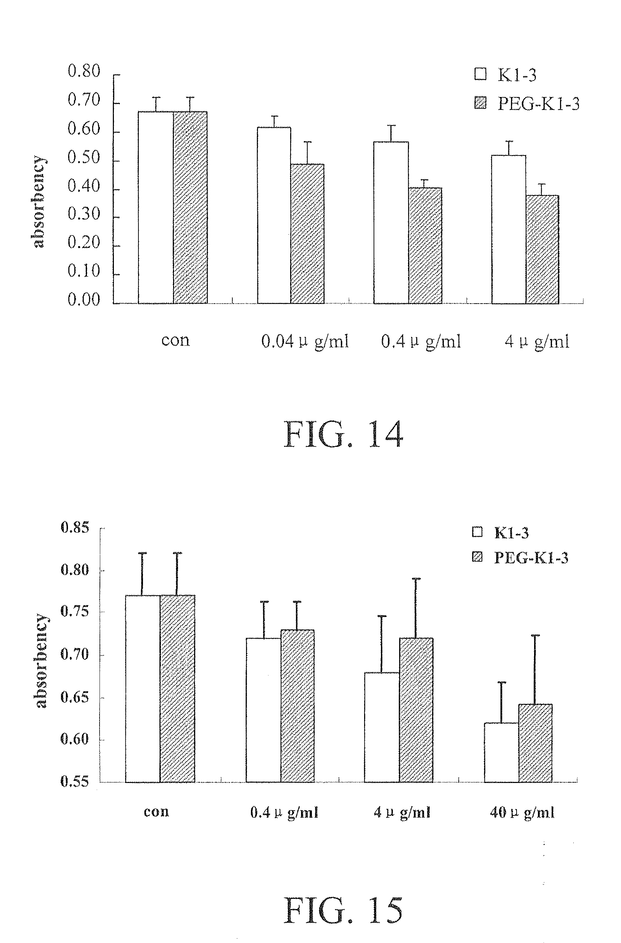

The K1-3 used in this example has the sequence of SEQ ID NO.2. HUVECs were starved overnight, inoculated in 96-well plates at the concentration of 2×10⁴/ml and cultured in the M199 medium (Hyclone) containing 20% fetal bovine serum (Hyclone) to exponential phase. After starved for 12 hours, cells were treated with K1-3 and K1-3 with PEG. Experimental groups were as follows: negative control (PBS), positive control (K1-3 at the concentration of 0.04, 0.4 and 4 µg/ml) and treatment group (K1-3 with PEG at the concentration of 0.04, 0.4 and 4 µg/ml). After culturing for 48 hours at 37 °C, cells were incubated with MTT (Amresco) for 4 hours at 37°C. Then DMSO (Shenggong, China) was added and absorption at 570 nm was measured by Microplate Reader (Model 550, Biorad). Results are indicated in Figure 14.

### Example 8: The activity of K1-3 with PEG coupled to its N-terminus of inhibiting the proliferation of vascular endothelial cells

K1-3 used in this example is the K1-3 having the sequence of SEQ ID NO.2. Specifically, the starved human microvascular endothelial cells (HMEC) were added into 96-well plates at 20, 000 cells per well, then cultured in the DMEM medium (Hyclone) containing 10% serum to exponential phase. After starving for 12 hours, cells were treated with different concentrations of modified or unmodified K1-3 for measurement of cell proliferation. Experimental groups were as follows: negative control group (PBS), positive control groups (0.04, 0.4, and 4 µg/ml K1-3), and treatment group (0.04, 0.4, and 4 µg/ml K1-3 with PEG coupled to its N-terminus). After culturing for 48 hours at 37 °C, the cells in the plates were treated with MTT for 4 h at 37 °C, Then DMSO was added, and the absorption was measured at 570 nm by microplate spectrophotometer. The experimental results are shown in Figure 15.

### Example 9: The activity of K1-3 with PEG coupled to its N-terminus of inhibiting the proliferation of tumor cells (human cervical cancer, HeLa)

K1-3 used in this example is the K1-3 having the sequence of SEQ ID NO.2. Specifically, HeLa (ATCC# CCL-2, USA) were added into 96-well plates at 20, 000 cells per well, then cultured in the DMEM medium (Hyclone) containing 10% serum to exponential phase. After starving for 12 hours, cells were treated with different concentrations of modified or unmodified K1-3 for measurement of cell proliferation. Experimental groups were as follows: negative control group (PBS), positive control groups (0.4, 4, 40, and 150 µg/ml K1-3), and treatment group (0.4, 4, 40, and 150 µg/ml K1-3 with PEG coupled to its N-terminus). After culturing for 48 hours at 37 °C, the cells in the plates were treated with MTT for 4 h at 37 °C. Then DMSO was added, and the absorption was measured at 570 nm by microplate spectrophotometer. The results revealed that K1-3 with PEG coupled to its N-terminus was more potent than unmodified K1-3 in the inhibition of the tumor cell proliferation. The results also showed that, compared with the concentrations of 0.4, 4, and 150 µg/ml, K1-3 and K1-3 with PEG coupled to its N-terminus showed the highest anti-proliferation effect on tumor cells at the concentration of 40 µg/ml. The experimental results are shown in Figure 16.

### Example 10: Activity of K1-3 with PEG coupled to its N-terminus in the treatment of late-stage B16 melanoma mice tumor model.

K1-3 used in this example is K1-3 having the sequence of SEQ ID NO.2. Experiments were performed to observe the *in vivo* inhibitory activity of K1-3 with PEG coupled to its N-terminus on late-stage B16 tumor. C57B/L mice of about 20 g were used in the experiment (Beijing Vital River Laboratory Animal Co. Ltd.). Mice were inoculated at back with B16 cells (ATCC# CRL-6475, USA), 1 x 106 cells per mouse. The mice were then randomly grouped after the tumor volume reaches 1 cm³, 8 mice per group, including the negative control group (sodium acetate), the positive control group (K1-3, 4.5 mg/kg body weight, administered daily), three treatment groups (K1-3 with PEG coupled to its N-terminus, 1.5 mg/kg body weight, administered daily, or 4.5 mg/kg body weight, administered daily, every 3 days, every 6 days, or every 9 days, respectively). Administration was performed by subcutaneous injection and lasted for 10 days. The mice were sacrificed on day 11, the weight of tumor was measured, and the efficacy was evaluated by tumor inhibition rate. For K1-3 having the sequence of SEQ ID NO.2with PEG coupled to its N-terminus, the experimental results revealed that the tumor inhibition rate of the positive control group and the groups treated with 1.5 mg/kg body weight daily was 45% and 28%, respectively, whereas the tumor inhibition rates were 60%, 34%, 25%, and 24% for the groups treated with 4.5 mg/kg body weight, daily, every 3 days, every 6 days, or every 9 days, respectively. The results demonstrate that the anti-tumor activity of K1-3 with PEG coupled to its N-terminus is superior to that of the unmodified K1-3. Furthermore, it can maintain its superior efficacy even when administered at a low concentration. The experimental results are shown in Figure 17.

### Example 11: Activity of K1-3 with PEG coupled to its N-terminus in the treatment of early-stage H22 hepatocellular carcinoma mice tumor model.

K1-3 used in this example is K1-3 having the sequence of SEQ ID NO.2. Experiments were performed to observe the *in vivo* inhibitory activity of K1-3 with PEG coupled to its N-terminus on early-stage H22 tumor. Balb/c mice of about 20 g were used in the experiment (Beijing Vital River Laboratory Animal Co. Ltd.). Mice were inoculated at back with H22 cells, 1 x 10⁶ cells per mouse. The mice were then randomly grouped after the tumor volume reaches 1 cm³, 8 mice per group, including the negative control group (sodium acetate), the positive control group (K1-3, 4.5 mg/kg body weight, administered daily), three treatment groups (K1-3 with PEG coupled to its N-terminus, 1.5 mg/kg body weight, administered daily, or 4.5 mg/kg body weight, administered daily, twice per week, or weekly, respectively). The administration was performed by subcutaneous injection and lasted for 3 weeks. The mice were sacrificed on day 22, the weight of tumor was measured, and the efficacy was evaluated by tumor inhibition rate. For K1-3 having the sequence of SEQ ID NO.2 with PEG coupled to its N-terminus , the experimental results revealed that the tumor inhibition rate of the positive control group and the groups treated with 1.5 mg/kg body weight daily was 51% and 54%, respectively, whereas the tumor inhibition rates were 60%, 84%, and 50% for the groups treated with 4.5 mg/kg body weight, daily, twice per week, and weekly, respectively. The results demonstrate that the anti-tumor activity of K1-3 with PEG coupled to its N-terminus is superior to that of the unmodified K1-3. Furthermore, the efficacy of K1-3 at a low concentration is superior in inhibiting the growth of liver cancer cells than that of K1-3 at a higher concentration. Since the *in vivo* half life of K1-3 with PEG coupled to its N-terminus are longer than that of unmodified K1-3 of the same dosage, the biological activity K1-3 with PEG coupled to its N-terminus is higher than that of the unmodified K1-3 of the same concentration when administration intervals are extended. Administrations at twice per week showed the highest anti-tumor activity, up to more than 80%. The experimental results are shown in Figure 18.

### Example 12: The in vivo anti-tumor activity of a conjugate of PEG and K1-3 (PEG-K1-3) in NCI-H226 NSCLC xenograft model.

The recombinant protein K1-3 used in this example has a sequence of SEQ ID NO:2. Female Balb/c nu/nu mice aged 6-8 weeks of about 18-20g weight (institute of laboratory animal sciences, CAMS) were used in this experiment. Mice were subcutaneously inoculated on the right side with NCI-H226 cells (human pancreatic cancer cell line, derived from ATCC, ATCC number CRL-5826). When the tumor volume reached about 80-125 mm³, the animals were divide into 3 groups randomly (6 per group). The treatment was administrated according to the following Table 1. The body weight and tumor volume of the animals were measured twice a week, Sacrifice the animals on day 28, collect the tumors and take photographs.

**Table 1: Grouping of animals and the corresponding treatments.**

| Group | Sample size | drug name | dosage | drug volume | administration route | administration time |
|---|---|---|---|---|---|---|
| 1 | 6 | Vector | 0 mg/kg | 5 µl/g | i.v. | Twice a week * 3 weeks |
| 2 | 6 | PEG-K1-3 | 4.5 mg/kg | 5 µl/g | i.v. | Twice a week * 3 weeks |
| 3 | 6 | Taxol | 20 mg/kg | 10 µl/g | i.v. | Twice a week * 3 weeks |

| | | | | | | |
|---|---|---|---|---|---|---|
| Remark: Taxol was purchased from Beijing Union Pharmaceutical Factory. Lot number 060408. | | | | | | |

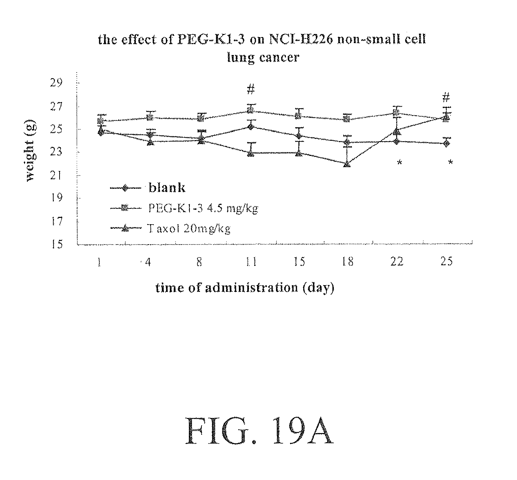

The results revealed that, as is indicated in Fig 19A, on Day 22 and Day25, the average body weight of the animals in the group treated with 4.5 mg/kg PEG-K1-3 (Group 2) is significantly different from that of the animals in the control group (Group 1). The body weights of Group 2 are significantly heavier than that of Group 1, single variant ANOVA, *p<0.05. On Day 11 and Day 25, the average body weight of the animals in the group treated with 20 mg/kg taxol (Group 3) is significantly different from that of the animals in the control group. The average body weight of animals in Group 3 is significantly lower than that of the animals in Group 1, single variant ANOVA, *p<0.05.

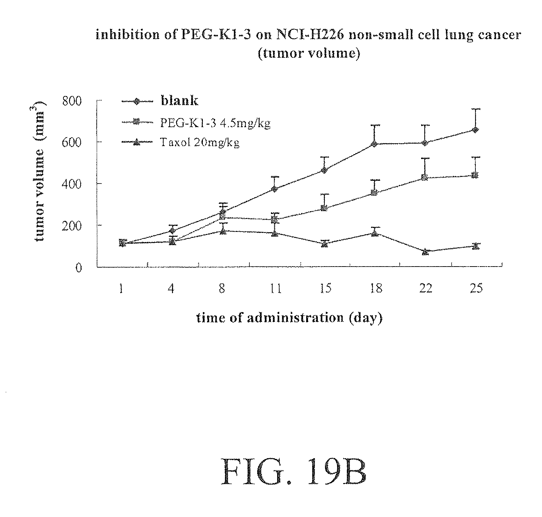

As is shown in Figure 19B, compared with the control group, tumor burden of those animals treated with 4.5 mg/kg PEG-K1-3 (Group 2) is significantly decreased (p<0.05). Tumor burden of animals in Group 3 show significantly difference in Day 11, Day 15, Day 18. Furthermore, in Day 4, Day 11, Day 18 and Day 25, the T/C ratios of Group 2 (4.5m.g/kg PEG-K1-3 i.v. * 21 days) are 73%, 63%, 62% and 66%, respectively. While T/C ratios of Group 3 (20mg/kg Taxol) are 69%, 39%, 28% and 18%, respectively.

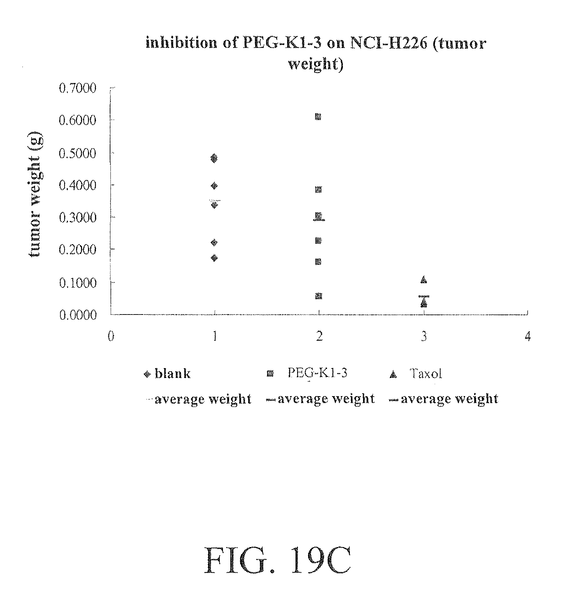

As is shown in Figure 19C, the tumor burden 21 days after treatment is calculated. Compared to the control group (Group 1), tumor inhibition rate in animals treated with 4.5 mg/kg PEG-K1-3 (Group 2) and animals treated with 20mg/kg Taxol (Group 3) are 17.18% and 84.43%, respectively. Tumor burden in animals treated with 4.5 mg/kg PEG-K1-3 and the animals in the control group show significant difference compared to animals treated with Taxol. (P=0.008 and P=0.026, respectively)

## Claims

1. A conjugate of polyethylene glycol and angiostatin fragment K1-3, wherein the conjugate exhibits prolonged *in vivo* half-life and enhanced biological activity as compared to unmodified angiostatin fragment K1-3, and wherein the conjugate is formed by coupling one angiostatin fragment K1-3 with one polyethylene glycol molecule at the N-terminal α-amino group of the angiostatin fragment K1-3, and said angiostatin fragment K1-3 consists of a sequence as shown in SEQ ID NO:1, or a sequence having at least 80% amino acid sequence homology to SEQ ID NO:1, and wherein said polyethylene glycol molecule has a molecular weight of 5,000 to 40,000 Daltons.

2. The conjugate of claim 1, wherein said angiostatin fragment K1-3 is a recombinant angiostatin fragment K1-3 preferably a recombinant human angiostatin fragment K1-3 consisting of a sequence as shown in SEQ ID NO:2.

3. The conjugate of claim 1, wherein said polyethylene glycol is monomethyl polyethylene glycol.

4. The conjugate of claim 3, wherein said monomethyl polyethylene glycol has a molecular weight of 20 kDa.

5. The conjugate of any one of claims 1 to 4, wherein said polyethylene glycol is linear or branched.

6. The conjugate of any one of claims 1 to 5, wherein said polyethylene glycol molecule is monomethoxy polyethylene glycol butyraldehyde molecule of 20 kDa.

7. A sustained-release formulation comprising the conjugate of any one of claims 1 to 6 and a bio-compatible substance, wherein the sustained-release formulation is in a form selected from the group consisting of a microcapsule, a hydrogel, a microsphere, a micro-osmotic pump and a liposome.

8. A pharmaceutical composition comprising the conjugate of any one of claims 1 to 6 or the sustained-release formulation of claim 7 and a pharmaceutically acceptable carrier.

9. The conjugate of any one of claims 1 to 6, the sustained-release formulation of claim 7 or the pharmaceutical composition of claim 8 for use as a medicament.

10. Use of the conjugate of any one of claims 1 to 6, the sustained-release formulation of claim 7 or the pharmaceutical composition of claim 8 in the preparation of medicaments for treating tumor diseases or medicaments for treating non-tumor diseases **characterized by** pathological changes in human tissues or organs caused by abnormal neovascularization.

11. The use of claim 10, wherein the tumor disease is selected from a group consisting of lung cancer, neuroendocrine tumor, colon cancer, bone cancer, liver cancer, gastric cancer, pancreatic cancer, oral cancer, breast cancer, prostate cancer, lymphoma, esophagus cancer, nasopharyngeal carcinoma, cervical cancer, sarcoma, renal carcinoma, biliary cancer, and malignant melanoma.

12. A method to prolong the *in vivo* half-life and enhance biological activity of the angiostatin fragment K1-3, comprising the steps of forming the conjugate of any one of claims 1-6 by using angiostatin fragment K1-3 and polyethylene glycol.

## Patentansprüche

1. Konjugat von Polyethylenglykol und Angiostatin-Fragment K1-3, wobei das Konjugat eine verlängerte in vivo-Halbwertszeit und eine erhöhte biologische Aktivität im Vergleich zu einem nicht-modifizierten Angiostatin-Fragment K1-3 aufweist und wobei das Konjugat durch Kupplung eines Angiostatin-Fragments K1-3 mit einem Polyethylenglykol-Molekül an der N-terminalen α-Aminogruppe des Angiostatin-Fragments K1-3 gebildet wird und das Angiostatin-Fragment K1-3 aus einer Sequenz, wie in SEQ ID NO: 1 gezeigt, oder einer Sequenz mit zumindest 80 %-iger Aminosäuresequenzhomologie zu SEQ ID NO: 1 besteht, und wobei das Polyethylenglykol-Molekül ein Molekulargewicht von 5.000 bis 40.000 Dalton aufweist.

2. Konjugat gemäss Anspruch 1, wobei das Angiostatin-Fragment K1-3 ein rekombinantes Angiostatin-Fragment K1-3, vorzugsweise ein rekombinantes humanes Angiostatin-Fragment K1-3, bestehend aus einer Sequenz, wie in SEQ ID NO: 2 gezeigt, ist.

3. Konjugat gemäss Anspruch 1, wobei das Polyethylenglykol Monomethylpolyethylenglykol ist.

4. Konjugat gemäss Anspruch 3, wobei das Monomethylpolyethylenglykol ein Molekulargewicht von 20 kDa aufweist.

5. Konjugat gemäss irgendeinem der Ansprüche 1 bis 4, wobei das Polyethylenglykol geradkettig oder verzweigt ist.

6. Konjugat gemäss irgendeinem der Ansprüche 1 bis 5, wobei das Polyethylenglykol-Molekül ein Monomethoxypolyethylenglykolbutyraldehyd-Molekül mit 20 kDa ist.

7. Formulierung mit verzögerter Freisetzung, umfassend das Konjugat gemäss irgendeinem der Ansprüche 1 bis 6 und eine biokompatible Substanz, wobei die Formulierung mit verzögerter Freisetzung in einer Form vorliegt, die aus der Gruppe bestehend aus einer Mikrokapsel, einem Hydrogel, einem Mikrokügelchen, einer mikroosmotischen Pumpe und einem Liposom ausgewählt ist.

8. Pharmazeutische Zusammensetzung, umfassend das Konjugat gemäss irgendeinem der Ansprüche 1 bis 6 oder die Formulierung mit verzögerter Freisetzung gemäss Anspruch 7 und einen pharmazeutisch annehmbaren Träger.

9. Konjugat gemäss irgendeinem der Ansprüche 1 bis 6, die Formulierung mit verzögerter Freisetzung gemäss Anspruch 7 oder die pharmazeutische Zusammensetzung gemäss Anspruch 8 zur Verwendung als Arzneimittel.

10. Verwendung des Konjugats gemäss irgendeinem der Ansprüche 1 bis 6, der Formulierung mit verzögerter Freisetzung gemäss Anspruch 7 oder der pharmazeutischen Zusammensetzung gemäss Anspruch 8 bei der Herstellung eines Arzneimittels zur Behandlung von Tumorerkrankungen oder eines Arzneimittels zur Behandlung von Nicht-Tumorerkrankungen, die durch pathologische Veränderungen in menschlichen Geweben oder Organen, verursacht durch abnormale Neovaskularisierung, gekennzeichnet sind.

11. Verwendung gemäss Anspruch 10, wobei die Tumorerkrankung aus der Gruppe bestehend aus Lungenkrebs, neuroendokrinem Tumor, Darmkrebs, Knochenkrebs, Leberkrebs, Magenkrebs, Bauchspeicheldrüsenkrebs, Mundhöhlenkrebs, Brustkrebs, Prostatakrebs, Lymphom, Speiseröhrenkrebs, Nasopharynxkarzinom, Gebärmutterhalskrebs, Sarkom, Nierenkarzinom, Gallenkrebs und malignem Melanom ausgewählt ist.

12. Verfahren zur Verlängerung der in vivo-Halbwertszeit und Erhöhung der biologischen Wirkung des Angiostatin-Fragments K1-3, umfassend die Schritte: Bilden des Konjugats gemäss irgendeinem der Ansprüche 1 bis 6 unter Verwendung des Angiostatin-Fragments K1-3 und Polyethylenglykol.

## Revendications

1. Conjugué de polyéthylèneglycol et d'un fragment d'angiostatine K1-3, dans lequel le conjugué montre une demi-vie *in vivo* prolongée et une activité biologique améliorée en comparaison d' un fragment d'angiostatine K1-3 non-modifié, dans lequel le conjugué est formé en couplant un fragment d'angiostatine K1-3 avec une molécule de polyéthylèneglycol au groupement α-amino N-terminal du fragment d'angiostatine K1-3 et ledit fragment d'angiostatine K1-3 est constitué d'une séquence telle que représentée dans la SEQ ID NO: 1 ou d'une séquence présentant au moins 80 % d'homologie de séquence d'acides aminés avec la SEQ ID NO: 1, et dans lequel ladite molécule de polyéthylèneglycol a un poids moléculaire de 5000 à 40 000 daltons.

2. Conjugué selon la revendication 1, dans lequel ledit fragment d'angiostatine K1-3 est un fragment d'angiostatine K1-3 recombinant, de préférence un fragment d'angiostatine K1-3 recombinant constitué d'une séquence telle que représentée dans la SEQ ID NO: 2.

3. Conjugué selon la revendication 1, dans lequel ledit polyéthylèneglycol est un polyéthylèneglycol monométhylé.

4. Conjugué selon la revendication 3, dans lequel ledit polyéthylèneglycol monométhylé a un poids moléculaire de 20 kDa.

5. Conjugué selon l'une quelconque des revendications 1 à 4, dans lequel ledit polyéthylèneglycol est linéaire ou ramifié.

6. Conjugué selon l'une quelconque des revendications 1 à 5, dans lequel ladite molécule de polyéthylèneglycol est une molécule de butyraldéhyde de monométhoxy polyéthylèneglycol de 20 kDa.

7. Formulation à libération prolongée comprenant le conjugué selon l'une quelconque des revendications 1 à 6 et une substance biocompatible, dans laquelle la formulation à libération prolongée se présente sous une forme choisie dans le groupe constitué d'une microcapsule, d'un hydrogel, d'une microsphère, d'une pompe micro-osmotique et d'un liposome.

8. Composition pharmaceutique comprenant le conjugué selon l'une quelconque des revendications 1 à 6 ou la formulation à libération prolongée selon la revendication 7 et un véhicule pharmaceutiquement acceptable.

9. Conjugué selon l'une quelconque des revendications 1 à 6, formulation à libération prolongée selon la revendication 7 ou composition pharmaceutique selon la revendication 8 pour utilisation comme médicament.

10. Utilisation du conjugué selon l'une quelconque des revendications 1 à 6, de la formulation à libération prolongée selon la revendication 7 ou de la composition pharmaceutique selon la revendication 8 dans la préparation de médicaments destinés au traitement de maladies tumorales ou de médicaments destinés au traitement de maladies non tumorales **caractérisées par** des changements pathologiques dans des tissus ou des organes humains provoqués par une néovascularisation anormale.

11. Utilisation selon la revendication 10, dans laquelle la maladie tumorale est choisie dans le groupe consistant en un cancer du poumon, une tumeur neuroendocrinienne, un cancer du côlon, un cancer des os, un cancer du foie, un cancer de l'estomac, un cancer du pancréas, un cancer de la bouche, un cancer du sein, un cancer de la prostate, un lymphome, un cancer de l'oesophage, un carcinome nasopharyngien, un cancer du col de l'utérus, un sarcome, un carcinome rénal, un cancer des voies biliaires et un mélanome malin.

12. Procédé destiné à prolonger la demi-vie *in vivo* et à améliorer l'activité biologique du fragment d'angiostatine K1-3, comprenant les étapes qui consistent à former le conjugué selon l'une quelconque des revendications 1 à 6 en utilisant un fragment d'angiostatine K1-3 et du polyéthylèneglycol.


### Sequence Listing

<110> Protgen Ltd; Tsinghua University
<120> A conjugate comprising angiostatin or its fragment, the method for
   producing the conjugate and use thereof
<130> 162700
<150> CN2007 10004558
   <151> 2007-01-10
<160> 7
<170> PatentIn version 3.3
<210> 1
   <211> 250
   <212> PRT
   <213> Homo sapiens
<400> 1
   Cys Lys Thr Gly Asn Gly Lys Asn Tyr Arg Gly Thr Met Ser Lys Thr
   1               5                   10                  15
   Lys Asn Gly Ile Thr Cys Gln Lys Trp Ser Ser Thr Ser Pro His Arg
               20                  25                  30
   Pro Arg Phe Ser Pro Ala Thr His Pro Ser Glu Gly Leu Glu Glu Asn
           35                  40                  45
   Tyr Cys Arg Asn Pro Asp Asn Asp Pro Gln Gly Pro Trp Cys Tyr Thr
       50                  55                  60
   Thr Asp Pro Glu Lys Arg Tyr Asp Tyr Cys Asp Ile Leu Glu Cys Glu
   65                  70                  75                  80
   Glu Glu Cys Met His Cys Ser Gly Glu Asn Tyr Asp Gly Lys Ile Ser
                   85                  90                  95
   Lys Thr Met Ser Gly Leu Glu Cys Gln Ala Trp Asp Ser Gln Ser Pro
               100                 105                 110
   His Ala His Gly Tyr Ile Pro Ser Lys Phe Pro Asn Lys Asn Leu Lys
           115                 120                 125
   Lys Asn Tyr Cys Arg Asn Pro Asp Arg Glu Leu Arg Pro Trp Cys Phe
       130                 135                 140
   Thr Thr Asp Pro Asn Lys Arg Trp Glu Leu Cys Asp Ile Pro Arg Cys
   145                 150                 155                 160
   Thr Thr Pro Pro Pro Ser Ser Gly Pro Thr Tyr Gln Cys Leu Lys Gly
                   165                 170                 175
   Thr Gly Glu Asn Tyr Arg Gly Asn Val Ala Val Thr Val Ser Gly His
               180                 185                 190
   Thr Cys Gln His Trp Ser Ala Gln Thr Pro His Thr His Asn Arg Thr
           195                 200                 205
   Pro Glu Asn Phe Pro Cys Lys Asn Leu Asp Glu Asn Tyr Cys Arg Asn
       210                 215                 220
   Pro Asp Gly Lys Arg Ala Pro Trp Cys His Thr Thr Asn Ser Gln Val
   225                 230                 235                 240
   Arg Trp Glu Tyr Cys Lys Ile Pro Ser Cys
                   245                 250
<210> 2
   <211> 260
   <212> PRT
   <213> Homo sapiens
<400> 2
   Met Val Tyr Leu Ser Glu Cys Lys Thr Gly Asn Gly Lys Asn Tyr Arg
   1               5                   10                  15
   Gly Thr Met Ser Lys Thr Lys Asn Gly Ile Thr Cys Gln Lys Trp Ser
               20                  25                  30
   Ser Thr Ser Pro His Arg Pro Arg Phe Ser Pro Ala Thr His Pro Ser
           35                  40                  45
   Glu Gly Leu Glu Glu Asn Tyr Cys Arg Asn Pro Asp Asn Asp Pro Gln
       50                  55                  60
   Gly Pro Trp Cys Tyr Thr Thr Asp Pro Glu Lys Arg Tyr Asp Tyr Cys
   65                  70                  75                  80
   Asp Ile Leu Glu Cys Glu Glu Glu Cys Met His Cys Ser Gly Glu Asn
                   85                  90                  95
   Tyr Asp Gly Lys Ile Ser Lys Thr Met Ser Gly Leu Glu Cys Gln Ala
               100                 105                 110
   Trp Asp Ser Gln Ser Pro His Ala His Gly Tyr Ile Pro Ser Lys Phe
           115                 120                 125
   Pro Asn Lys Asn Leu Lys Lys Asn Tyr Cys Arg Asn Pro Asp Arg Glu
       130                 135                 140
   Leu Arg Pro Trp Cys Phe Thr Thr Asp Pro Asn Lys Arg Trp Glu Leu
   145                 150                 155                 160
   Cys Asp Ile Pro Arg Cys Thr Thr Pro Pro Pro Ser Ser Gly Pro Thr
                   165                 170                 175
   Tyr Gln Cys Leu Lys Gly Thr Gly Glu Asn Tyr Arg Gly Asn Val Ala
               180                 185                 190
   Val Thr Val Ser Gly His Thr Cys Gln His Trp Ser Ala Gln Thr Pro
           195                 200                 205
   His Thr His Asn Arg Thr Pro Glu Asn Phe Pro Cys Lys Asn Leu Asp
       210                 215                 220
   Glu Asn Tyr Cys Arg Asn Pro Asp Gly Lys Arg Ala Pro Trp Cys His
   225                 230                 235                 240
   Thr Thr Asn Ser Gln Val Arg Trp Glu Tyr Cys Lys Ile Pro Ser Cys
                   245                 250                 255
   Asp Ser Ser Pro
               260
<210> 3
   <211> 79
   <212> PRT
   <213> Homo sapiens
<400> 3
   Cys Lys Thr Gly Asn Gly Lys Asn Tyr Arg Gly Thr Met Ser Lys Thr
   1               5                   10                  15
   Lys Asn Gly Ile Thr Cys Gln Lys Trp Ser Ser Thr Ser Pro His Arg
               20                  25                  30
   Pro Arg Phe Ser Pro Ala Thr His Pro Ser Glu Gly Leu Glu Glu Asn
           35                  40                  45
   Tyr Cys Arg Asn Pro Asp Asn Asp Pro Gln Gly Pro Trp Cys Tyr Thr
       50                  55                  60
   Thr Asp Pro Glu Lys Arg Tyr Asp Tyr Cys Asp Ile Leu Glu Cys
   65                  70                  75
<210> 4
   <211> 78
   <212> PRT
   <213> Homo sapiens
<400> 4
   Cys Met His Cys Ser Gly Glu Asn Tyr Asp Gly Lys Ile Ser Lys Thr
   1               5                   10                  15
   Met Ser Gly Leu Glu Cys Gln Ala Trp Asp Ser Gln Ser Pro His Ala
               20                  25                  30
   His Gly Tyr Ile Pro Ser Lys Phe Pro Asn Lys Asn Leu Lys Lys Asn
           35                  40                  45
   Tyr Cys Arg Asn Pro Asp Arg Glu Leu Arg Pro Trp Cys Phe Thr Thr
       50                  55                  60
   Asp Pro Asn Lys Arg Trp Glu Leu Cys Asp Ile Pro Arg Cys
   65                  70                  75
<210> 5
   <211> 78
   <212> PRT
   <213> Homo sapiens
<400> 5
   Cys Leu Lys Gly Thr Gly Glu Asn Tyr Arg Gly Asn Val Ala Val Thr
   1               5                   10                  15
   Val Ser Gly His Thr Cys Gln His Trp Ser Ala Gln Thr Pro His Thr
               20                  25                  30
   His Asn Arg Thr Pro Glu Asn Phe Pro Cys Lys Asn Leu Asp Glu Asn
           35                  40                  45
   Tyr Cys Arg Asn Pro Asp Gly Lys Arg Ala Pro Trp Cys His Thr Thr
       50                  55                  60
   Asn Ser Gln Val Arg Trp Glu Tyr Cys Lys Ile Pro Ser Cys
   65                  70                  75
<210> 6
   <211> 266
   <212> PRT
   <213> Homo sapiens
<400> 6
   Met His His His His His His Val Tyr Leu Ser Glu Cys Lys Thr Gly
   1               5                   10                  15
   Asn Gly Lys Asn Tyr Arg Gly Thr Met Ser Lys Thr Lys Asn Gly Ile
               20                  25                  30
   Thr Cys Gln Lys Trp Ser Ser Thr Ser Pro His Arg Pro Arg Phe Ser
           35                  40                  45
   Pro Ala Thr His Pro Ser Glu Gly Leu Glu Glu Asn Tyr Cys Arg Asn
       50                  55                  60
   Pro Asp Asn Asp Pro Gln Gly Pro Trp Cys Tyr Thr Thr Asp Pro Glu
   65                  70                  75                  80
   Lys Arg Tyr Asp Tyr Cys Asp Ile Leu Glu Cys Glu Glu Glu Cys Met
                   85                  90                  95
   His Cys Ser Gly Glu Asn Tyr Asp Gly Lys Ile Ser Lys Thr Met Ser
               100                 105                 110
   Gly Leu Glu Cys Gln Ala Trp Asp Ser Gln Ser Pro His Ala His Gly
           115                 120                 125
   Tyr Ile Pro Ser Lys Phe Pro Asn Lys Asn Leu Lys Lys Asn Tyr Cys
       130                 135                 140
   Arg Asn Pro Asp Arg Glu Leu Arg Pro Trp Cys Phe Thr Thr Asp Pro
   145                 150                 155                 160
   Asn Lys Arg Trp Glu Leu Cys Asp Ile Pro Arg Cys Thr Thr Pro Pro
                   165                 170                 175
   Pro Ser Ser Gly Pro Thr Tyr Gln Cys Leu Lys Gly Thr Gly Glu Asn
               180                 185                 190
   Tyr Arg Gly Asn Val Ala Val Thr Val Ser Gly His Thr Cys Gln His
           195                 200                 205
   Trp Ser Ala Gln Thr Pro His Thr His Asn Arg Thr Pro Glu Asn Phe
       210                 215                 220
   Pro Cys Lys Asn Leu Asp Glu Asn Tyr Cys Arg Asn Pro Asp Gly Lys
   225                 230                 235                 240
   Arg Ala Pro Trp Cys His Thr Thr Asn Ser Gln Val Arg Trp Glu Tyr
                   245                 250                 255
   Cys Lys Ile Pro Ser Cys Asp Ser Ser Pro
   260 265
<210> 7
   <211> 715
   <212> PRT
   <213> Homo sapiens
<400> 7
   Met Pro Ser Thr Ser Phe Pro Val Pro Ser Lys Phe Pro Leu Gly Pro
   1               5                   10                  15
   Ala Ala Ala Val Phe Gly Arg Gly Glu Thr Leu Gly Pro Ala Pro Arg
               20                  25                  30
   Ala Gly Gly Thr Lys Ser Ala Glu Glu Glu His Tyr Gly Tyr Ala Ser
           35                  40                  45
   Ser Asn Val Ser Pro Ala Leu Pro Leu Pro Thr Ala His Ser Thr Leu
       50                  55                  60
   Pro Ala Pro Cys His Asn Leu Gln Thr Ser Thr Pro Gly Ile Ile Pro
   65                  70                  75                  80
   Pro Ala Asp His Pro Ser Gly Tyr Gly Ala Ala Leu Asp Gly Gly Pro
                   85                  90                  95
   Ala Gly Tyr Phe Leu Ser Ser Gly His Thr Arg Pro Asp Gly Ala Pro
               100                 105                 110
   Ala Leu Glu Ser Pro Arg Ile Glu Ile Thr Ser Cys Leu Gly Leu Tyr
           115                 120                 125
   His Asn Asn Asn Gln Phe Phe His Asp Val Glu Val Glu Asp Val Leu
       130                 135                 140
   Pro Ser Ser Lys Arg Ser Pro Ser Thr Ala Thr Leu Ser Leu Pro Ser
   145                 150                 155                 160
   Leu Glu Ala Tyr Arg Asp Pro Ser Cys Leu Ser Pro Ala Ser Ser Leu
                   165                 170                 175
   Ser Ser Arg Ser Cys Asn Ser Glu Ala Ser Ser Tyr Glu Ser Asn Tyr
               180                 185                 190
   Ser Tyr Pro Tyr Ala Ser Pro Gln Thr Ser Pro Trp Gln Ser Pro Cys
           195                 200                 205
   Val Ser Pro Lys Thr Thr Asp Pro Glu Glu Gly Phe Pro Arg Gly Leu
       210                 215                 220
   Gly Ala Cys Thr Leu Leu Gly Ser Pro Arg His Ser Pro Ser Thr Ser
   225                 230                 235                 240
   Pro Arg Ala Ser Val Thr Glu Glu Ser Trp Leu Gly Ala Arg Ser Ser
                   245                 250                 255
   Arg Pro Ala Ser Pro Cys Asn Lys Arg Lys Tyr Ser Leu Asn Gly Arg
               260                 265                 270
   Gln Pro Pro Tyr Ser Pro His His Ser Pro Thr Pro Ser Pro His Gly
           275                 280                 285
   Ser Pro Arg Val Ser Val Thr Asp Asp Ser Trp Leu Gly Asn Thr Thr
       290                 295                 300
   Gln Tyr Thr Ser Ser Ala Ile Val Ala Ala Ile Asn Ala Leu Thr Thr
   305                 310                 315                 320
   Asp Ser Ser Leu Asp Leu Gly Asp Gly Val Pro Val Lys Ser Arg Lys
                   325                 330                 335
   Thr Thr Leu Glu Gln Pro Pro Ser Val Ala Leu Lys Val Glu Pro Val
               340                 345                 350
   Gly Glu Asp Leu Gly Ser Pro Pro Pro Pro Ala Asp Phe Ala Pro Glu
           355                 360                 365
   Asp Tyr Ser Ser Phe Gln His Ile Arg Lys Gly Gly Phe Cys Asp Gln
       370                 375                 380
   Tyr Leu Ala Val Pro Gln His Pro Tyr Gln Trp Ala Lys Pro Lys Pro
   385                 390                 395                 400
   Leu Ser Pro Thr Ser Tyr Met Ser Pro Thr Leu Pro Ala Leu Asp Trp
                   405                 410                 415
   Gln Leu Pro Ser His Ser Gly Pro Tyr Glu Leu Arg Ile Glu Val Gln
               420                 425                 430
   Pro Lys Ser His His Arg Ala His Tyr Glu Thr Glu Gly Ser Arg Gly
           435                 440                 445
   Ala Val Lys Ala Ser Ala Gly Gly His Pro Ile Val Gln Leu His Gly
       450                 455                 460
   Tyr Leu Glu Asn Glu Pro Leu Met Leu Gln Leu Phe Ile Gly Thr Ala
   465 470 475 480
   Asp Asp Arg Leu Leu Arg Pro His Ala Phe Tyr Gln Val His Arg Ile
   485 490 495
   Thr Gly Lys Thr Val Ser Thr Thr Ser His Glu Ala Ile Leu Ser Asn
   500 505 510
   Thr Lys Val Leu Glu Ile Pro Leu Leu Pro Glu Asn Ser Met Arg Ala
   515 520 525
   Val Ile Asp Cys Ala Gly Ile Leu Lys Leu Arg Asn Ser Asp Ile Glu
   530 535 540
   Leu Arg Lys Gly Glu Thr Asp Ile Gly Arg Lys Asn Thr Arg Val Arg
   545 550 555 560
   Leu Val Phe Arg Val His Val Pro Gln Pro Ser Gly Arg Thr Leu Ser
   565 570 575
   Leu Gln Val Ala Ser Asn Pro Ile Glu Cys Ser Gln Arg Ser Ala Gln
   580 585 590
   Glu Leu Pro Leu Val Glu Lys Gln Ser Thr Asp Ser Tyr Pro Val Val
   595 600 605
   Gly Gly Lys Lys Met Val Leu Ser Gly His Asn Phe Leu Gln Asp Ser
   610 615 620
   Lys Val Ile Phe Val Glu Lys Ala Pro Asp Gly His His Val Trp Glu
   625 630 635 640
   Met Glu Ala Lys Thr Asp Arg Asp Leu Cys Lys Pro Asn Ser Leu Val
   645 650 655
   Val Glu Ile Pro Pro Phe Arg Asn Gln Arg Ile Thr Ser Pro Val His
   660 665 670
   Val Ser Phe Tyr Val Cys Asn Gly Lys Arg Lys Arg Ser Gln Tyr Gln
   675 680 685
   Arg Phe Thr Tyr Leu Pro Ala Asn Gly Asn Ala Ile Phe Leu Thr Val
   690 695 700
   Ser Arg Glu His Glu Arg Val Gly Cys Phe Phe
   705 710 715